(19) **Europäisches Patentamt / European Patent Office / Office européen des brevets**

(11) **EP 2 821 804 A1**

(12) # EUROPEAN PATENT APPLICATION

(43) Date of publication:
**07.01.2015 Bulletin 2015/02**

(51) Int Cl.:
***G01R 33/46*** *(2006.01)*

(21) Application number: **14153816.5**

(22) Date of filing: **04.02.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **05.07.2013 KR 20130079038**

(71) Applicant: **Samsung Electronics Co., Ltd Gyeonggi-do 443-742 (KR)**

(72) Inventors:
- **Cho, Hyug-rae**
  **Seoul (KR)**
- **Kim, Hei-soog**
  **Gyeonggi-do (KR)**
- **Park, Seon-mi**
  **Seoul (KR)**

(74) Representative: **D'Halleweyn, Nele Veerle Trees Gertrudis et al**
  **Arnold & Siedsma**
  **Bezuidenhoutseweg 57**
  **2594 AC The Hague (NL)**

(54) **Apparatus and method of generating magnetic resonance spectrum**

(57)     A method generates a magnetic resonance (MR) spectrum by obtaining an MR echo signal from an object in response to a transmitted radio frequency (RF) signal that is emitted towards the object. The method extracts from the MR signal, a plurality of signals corresponding to a plurality of frequency ranges and adjusts a phase of at least one of the extracted plurality of signals in a time domain. A combination signal is generated by combining the plurality of signals including the at least one extracted phase adjusted signal and generates an MR spectrum of the object in response to the combination signal.

FIG. 1

## Description

CLAIM OF PRIORITY

This application claims the benefit of Korean Patent Application No. 10-2013-0079038, filed on July 5, 2013, in the Korean Intellectual Property Office, the disclosure of which is incorporated herein in its entirety by reference.

BACKGROUND

1. Technical Field

A system concerns generating an MR spectrum including information about a type and an amount of a metabolite included in a predetermined area of an object.

2. Description of the Related Art

A magnetic resonance (MR) spectrum illustrates a distribution of biochemical information or metabolites of a bodily tissue. When a radio frequency (RF) signal is emitted towards an object that is placed in a high magnetic field and then is turned off, an MR echo signal having a specific resonance frequency is emitted from a material included in the object, for example, hydrogen nuclei. Since hydrogen may be included in various types of metabolites, chemical shift which refers to variation in resonance frequency of an MR signal emitted from a hydrogen nucleus in response to a molecular structure of a metabolite. Accordingly, a user may quantize a type and an amount of a metabolite included in the object by observing a peak point of a specific frequency included in a frequency spectrum of the MR signal. A known MR spectrum obtained by using a magnetic resonance imaging (MRI) apparatus typically has a signal-to-noise ratio (SNR) lower than that of an MR spectrum obtained by using a nuclear magnetic resonance (NMR) apparatus, and it is difficult to obtain an accurate spectrum because many factors may degrade an MR spectrum signal obtained from an object.

SUMMARY

A system improves the accuracy of an MR spectrum obtained by using an MRI apparatus by generating a magnetic resonance (MR) spectrum which may accurately measure a type and an amount of a metabolite included in an object.

A method generates a magnetic resonance (MR) spectrum by obtaining an MR echo signal from an object in response to a transmitted radio frequency (RF) signal that is emitted towards the object. The method extracts from the MR signal, a plurality of signals corresponding to a plurality of frequency ranges and adjusts a phase of at least one of the extracted plurality of signals in a time domain. A combination signal is generated by combining the plurality of signals including the at least one extracted phase adjusted signal and generates an MR spectrum of the object in response to the combination signal.

In a feature, each of the plurality of frequency ranges individually correspond to individual resonance frequency ranges of a plurality of metabolites selected by a user and the extracting activity comprises extracting the plurality of signals by applying a singular value decomposition (SVD) algorithm to the MR signal in the time domain. The adjusting of the phase of the at least one of the plurality of signals comprises adjusting a second signal having a phase that is different from a phase of a first signal from among the plurality of signals in the time domain by time delaying the second signal relative to the first signal. Also the adjusting of the phase of the second signal comprises adjusting the phase of the second signal so that a phase difference between the phase of the first signal and the phase of the second signal lies within a predetermined range. Further, adjusting of the phase of the second signal comprises adjusting the phase of the second signal so that the phase of the second signal is substantially the same as the phase of the first signal and the adjusting of the phase of the at least one of the plurality of signals comprises selecting from the plurality of signals, a signal having a phase of 0° as the first signal.

In another feature the method includes generating a second combination signal by combining a difference signal obtained by removing the extracted plurality of signals from the MR signal with the combination signal; and generating the MR spectrum of the object based on the second combination signal. The generating of the second combination signal comprises generating the second combination signal by combining a signal obtained by removing a signal corresponding to a resonance frequency range of water from the difference signal with the combination signal. Also the method includes generating the combination signal in the time domain, converting the combination signal to the frequency domain and generating the MR spectrum from the combination signal in the frequency domain and outputting the generated MR spectrum. A computer-readable recording medium has embodied thereon a program for executing the method.

In a further feature, an apparatus generates a magnetic resonance (MR) spectrum. The apparatus comprises an MR signal acquisition unit that obtains an MR echo signal from an object in response to a transmitted radio frequency (RF) signal that is emitted towards the object and a signal extracting unit that extracts from the MR signal, a plurality of signals corresponding to a plurality of frequency ranges. A signal control unit adjusts a phase of at least one of the extracted plurality of signals in a time domain, and generates a combination signal by combining the plurality of signals including the at least one extracted phase adjusted signal and a spectrum generating unit generates an MR spectrum of the object in response to the combination signal.

BRIEF DESCRIPTION OF THE DRAWINGS

These and/or other aspects will become apparent and more readily appreciated from the following description of the embodiments, taken in conjunction with the accompanying drawings of which:

> FIG. 1 shows a graph illustrating a known magnetic resonance (MR) spectrum and selection interface according to invention principles;
> FIG. 2 shows a detailed graph illustrating an MR spectrum that is generated from an MR signal obtained from an object according to invention principles;
> FIG. 3A is a graph illustrating a signal having a phase of 0° in a time domain and a frequency domain according to invention principles;
> FIG. 3B shows a graph illustrating a signal having a phase of 45° in a time domain and a frequency domain according to invention principles;
> FIG. 3C shows a graph illustrating a signal having a phase of 90° in a time domain and a frequency domain according to invention principles;
> FIG. 4 shows an apparatus for generating an MR spectrum, according to invention principles;
> FIG. 5 shows a graph illustrating a plurality of signals extracted from an MR signal in a time domain according to invention principles;
> FIG. 6 shows a graph illustrating a plurality of signals including a signal whose phase has been adjusted in a time domain according to invention principles;
> FIG. 7 shows an apparatus for generating an MR spectrum, according to invention principles;
> FIG. 8 shows a communication unit connected to the apparatus of FIG. 7 according to invention principles; and
> FIG. 9 shows a flowchart of a method of generating an MR spectrum, according to invention principles.

DETAILED DESCRIPTION

As used herein, the term "and/or" includes any and all combinations of one or more of the associated listed items. Expressions such as "at least one of," when preceding a list of elements, modify the entire list of elements and do not modify the individual elements of the list.

Terms used herein are terms known to one of ordinary skill in the art or are as defined herein to be interpreted in the context of description provided. Throughout the present application, when a part "includes" an element, it is to be understood that the part additionally includes other elements rather than excluding other elements as long as there is no particular opposing recitation. The term "unit" in the embodiments of the present invention means a software component or hardware component such as a processor, field-programmable gate array (FPGA) or an application-specific integrated circuit (ASIC), and performs a specific function. However, the term "unit" is not limited to software or hardware. The "unit" may be formed so as to be in an addressable storage medium, or may be formed so as to operate one or more processors. Thus, for example, the term "unit" may refer to components such as software components, object-oriented software components, class components, and task components, and may include processes, functions, attributes, procedures, subroutines, segments of program code, drivers, firmware, micro codes, circuits, data, a database, data structures, tables, arrays, or variables. A function provided by the components and "units" may be associated with the smaller number of components and "units", or may be divided into additional components and "units". Parts in the drawings unrelated to the detailed description are omitted to ensure clarity of the present invention.

The term "image" used herein may refer to multi-dimensional data composed of discrete image elements (for example, pixels for two-dimensional (2D) images and voxels for three-dimensional (3D) images). For example, the image may include a medical image of an object obtained by using an X-ray system, a computed tomography (CT) system, a magnetic resonance imaging (MRI) system, an ultrasonic system, or any other medical imaging system. Also, the term "object" used herein may include a human, an animal, or a body part of a human or an animal. For example, the object may include an organ such as the liver, heart, womb, brain, breast, or stomach, or a blood vessel. Also, the "object" may be a phantom made of a material having a volume very similar to an effective atomic number and a density of a living creature and having properties similar to those of a human body. Also, the term "user" used herein may refer to, but is not limited to, a medical expert such as a doctor, a nurse, a clinical pathologist, a medical image expert, or an engineer who repairs a medical device. The term "MRI image" used herein refers to an image of an object obtained by using nuclear magnetic resonance. The term "pulse sequence" used herein refers to a series of signals that are repeatedly applied in an MRI apparatus. The pulse sequence may have radio frequency (RF)-pulse time parameters such as a repetition time (TR) and an echo time (TE). Also, the term "pulse sequence diagram" used herein refers to a diagram illustrating a sequence of events that occur in an MRI apparatus. For example, the pulse sequence diagram may be a diagram illustrating an RF pulse, a gradient magnetic field, or a magnetic resonance (MR) signal according to a time line. The term "MR signal" used herein refers to an echo signal emitted from an object as a response to a radio frequency (RF) signal that is emitted towards the object. The MR signal includes signals emitted from a plurality of metabolites.

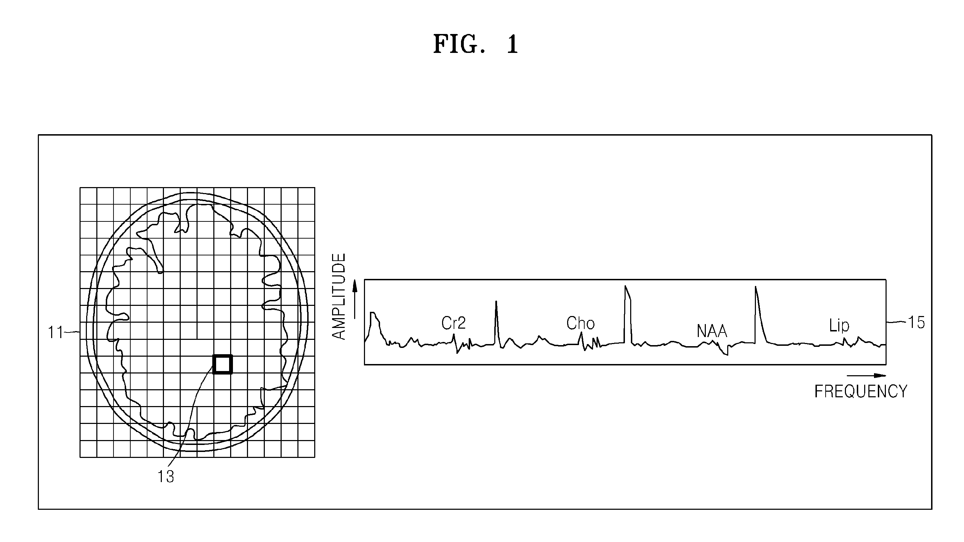

FIG. 1 shows a graph illustrating an MR spectrum 15 of a voxel of interest (VOI) of an MR image 11 of a brain of an object. When the VOI 13 included in the MR image 11 is selected by a user, the general MR spectrum 15 including information about a type and an amount of a metabolite included in the VOI 13 may be generated. The user may determine which metabolite is included in the VOI 13 and how much a metabolite is included in the VOI 13 by observing a peak point and a frequency of the peak point of the general MR spectrum 15.

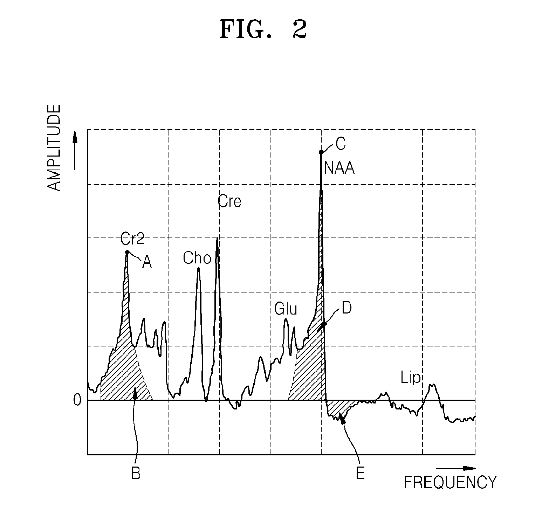

FIG. 2 shows a detailed graph illustrating an MR spectrum that is generated from an MR signal obtained from an object. A peak point A corresponds to a metabolite Cr2 and a peak point C corresponds to a metabolite NAA. A user may quantize an amount of the metabolite Cr2 by using an amplitude of the peak point A and a width of a peak area B corresponding to the peak point A, and may quantize an amount of the metabolite NAA by using an amplitude of the peak point C and a width of a peak area D+E corresponding to the peak point C. However, it is found that the peak area B corresponding to the peak point A does not include an area having an amplitude less than 0, whereas the peak area D+E corresponding to the peak area C includes an area E having an amplitude less than 0. When the peak area D+E corresponding to the metabolite NAA includes the area E having the amplitude less than 0, an amplitude of the peak point C and a width of the peak area D+E corresponding to the metabolite NAA may not be accurately measured, and thus it is difficult to accurately quantize a metabolite NAA. This is because when the peak area D+E corresponding to the metabolite NAA has an amplitude greater than 0, an accurate amplitude of the peak point C and an accurate width of the peak area D+E may not be calculated.

The reason why the peak area B corresponding to the metabolite Cr2 does not include an area having an amplitude less than 0 and the peak area D+E corresponding to the metabolite NAA includes the area E having an amplitude less than 0 is that a phase of a signal emitted from the metabolite Cr2 and a phase of a signal emitted from the metabolite NAA do not match to each other.

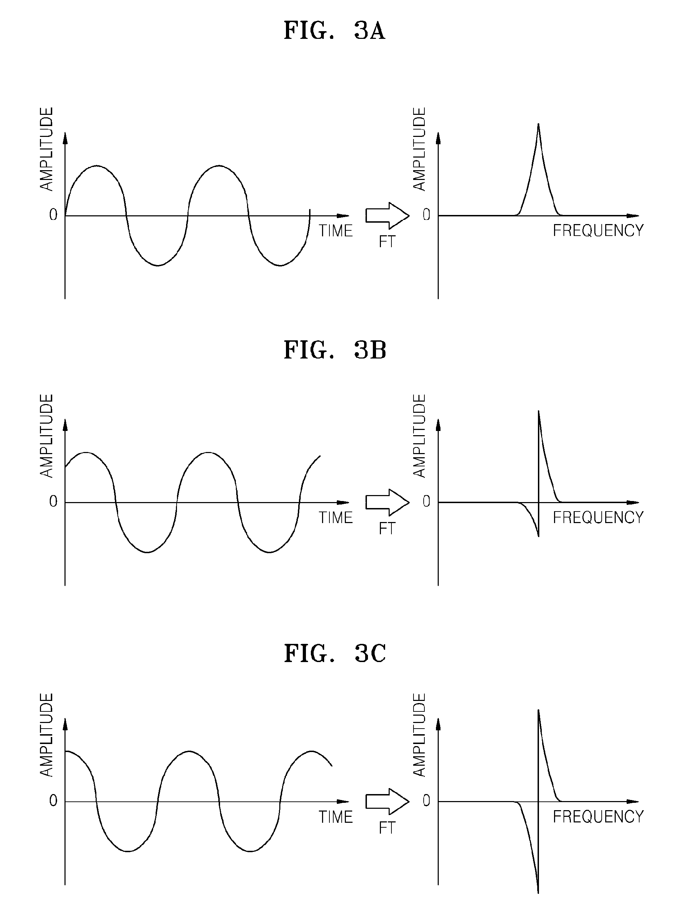

A relationship between a phase and a frequency spectrum of a signal is described with reference to FIGS. 3A, 3B, and 3C. FIG. 3A shows a graph illustrating a signal having a phase of 0° in a time domain and a frequency domain. FIG. 3B shows a graph illustrating a signal having a phase of 45° h a time domain and a frequency domain. FIG. 3C shows a graph illustrating a signal having a phase of 90° in a time domain and a frequency domain. The frequency spectrum of FIG. 3A does not include an area having an amplitude less than 0, whereas both the frequency spectra of FIG. 3B and FIG. 3C include an area having an amplitude less than 0.

Referring back to FIG. 2, a shape of the peak area B corresponding to the metabolite Cr2 is similar to the frequency spectrum of FIG. 3A and a shape of the peak area D+E corresponding to the metabolite NAA is different from the frequency spectrum of FIG. 3A. This indicates a signal emitted from the metabolite NAA is has different characteristics than a signal emitted from the metabolite Cr2. Factors that change phase of signal emitted from a plurality of metabolites are described.

There is a resonance frequency difference between the signals emitted from the plurality of metabolites, and a time difference between when a signal is emitted from each of the plurality of metabolites and when the emitted signal is received by an RF coil. As time increases between when a first signal having a relatively high resonance frequency and a second signal having a relatively low resonance frequency are emitted and when the first signal and the second signal are received by the RF coil, a phase difference between the first and second signals increases. A change in phase due to such a factor may be corrected by applying a predetermined amount of phase offset to an MR spectrum in a frequency domain or by linearly increasing or reducing a phase of a signal at a specific frequency.

A phase of a signal emitted from a metabolite is changed by an eddy current and irregularity of a main magnetic field in a gantry of an MRI apparatus. A change in a phase due to such a factor may not be corrected by applying a predetermined amount of phase offset to an MR spectrum in a frequency domain or linearly increasing or reducing a phase of a signal at a specific frequency as described above. This is because the eddy current and the irregularity of the main magnetic field make a nonlinear and irregular change in the signal emitted from the metabolite. A user manually corrects the MR spectrum in response to signal phase change due to an eddy current and irregularity in a main magnetic field. However, manual correction of an MR spectrum is subject to user error and judgment, so quantization of the metabolite may be inaccurate, and a user is presented with a burdensome task. The system may accurately quantize a metabolite by automatically adjusting a phase of a signal emitted from the metabolite.

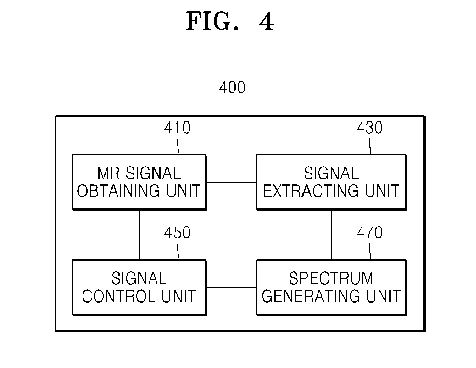

FIG. 4 shows apparatus 400 for generating an MR spectrum. Apparatus 400 may include an MR signal obtaining unit 410, a signal extracting unit 430, a signal control unit 450, and a spectrum generating unit 470. The MR signal obtaining unit 410, the signal extracting unit 430, the signal control unit 450, and the spectrum generating unit 470 may individually employ a microprocessor. The MR signal obtaining unit 410 obtains an echo MR signal that is emitted from an object as a response to a transmitted RF signal that is emitted towards the object. The MR signal may include a plurality of signals that are emitted from a plurality of metabolites included in the object. The MR signal obtaining unit 410 may acquire the MR signal through an RF coil that receives the MR signal emitted from the object. The MR signal obtained by the MR signal obtaining unit 410 may be an MR signal itself that is emitted from the object, or an MR signal on which pre-treatment such as zero-filling or filtering has been performed.

The signal extracting unit 430 extracts a plurality of signals respectively corresponding to a plurality of frequency ranges from the MR signal. Each of the plurality of frequency ranges may correspond to different individual resonance frequency ranges of the plurality of metabolites selected by the user. For example, when the user selects the metabolites NAA and Cr2 in order to determine amounts of the metabolites NAA and CR2 included in the object, the signal extracting unit 430 may extract from the MR signal a signal corresponding to a resonance frequency range emitted from the metabolite NAA and a signal corresponding to a resonance frequency range emitted from the metabolite Cr2.

The signal extracting unit 430 may extract the plurality of signals by applying a singular value decomposition (SVD) algorithm to the MR signal. In detail, the signal extracting unit 430 may divide the MR signal into signals corresponding to different frequency ranges by using an SVD algorithm, and may extract a plurality of signals corresponding to resonance frequency ranges selected by the user from among the divided signals. Alternatively, the signal extracting unit 430 may divide the MR signal into signals corresponding to different frequency ranges by using a Hankel singular value decomposition (HSVD) algorithm or another known method, and may extract a plurality of signals corresponding to resonance frequency ranges selected by the user from among the divided signals. For example, when the MR signal is M, the MR signal M may be divided by using an SVD algorithm as shown in Equation 1.

$$M = U \sum V^* \qquad \ldots (1).$$

In Equation 1, U is a left singular vector, $\Sigma$ is a singular value, and V is a right singular vector. In other words, the MR signal M may be expressed as a product of U, $\Sigma$, and V, and diagonal components of the singular value $\Sigma$ may be expressed as a singular value basis vector of the MR signal. The use of an SVD algorithm method for dividing a predetermined signal into signals corresponding to different frequency ranges is known so a detailed explanation thereof is omitted.

The signal control unit 450 adjusts a phase of at least one of the plurality of signals in a time domain, and generates a combination signal by combining the plurality of signals including the at least one phase adjusted signal. The signal control unit 450 may adjust a phase of a second signal having a phase that is different from a phase of a first signal. The spectrum generating unit 470 generates an MR spectrum of the object based on the combination signal. The spectrum generating unit 470 may generate the MR spectrum by converting the combination signal into a frequency domain. Since the apparatus 400 of FIG. 4 corrects the MR signal emitted from the object in a time domain and generates the MR spectrum based on the corrected MR signal, the user does not need to manually correct the MR spectrum and may accurately quantize a metabolite.

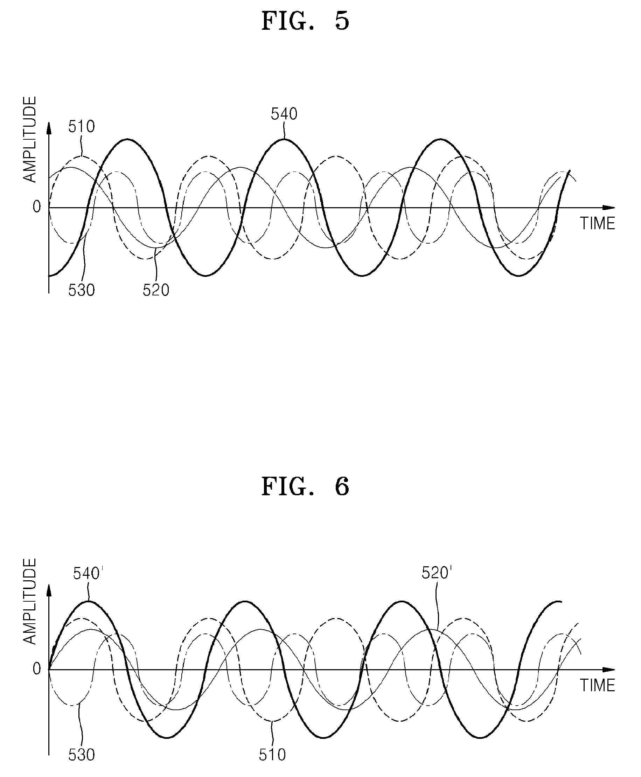

A method performed by the signal control unit 450 to adjust a phase of at least one of a plurality of signals in a time domain is described with reference to FIGS. 5 and 6.

FIG. 5 shows a graph illustrating a plurality of signals, for example, first through fourth signals 510, 520, 530, and 540, extracted from an MR signal in a time domain. Signals 510, 520, 530, and 540 of FIG. 5 respectively correspond to signals emitted from different metabolites included in an object. The first signal 510 and the third signal 530 of FIG. 5 has a phase of 0°, the second signal 520 has a phase of 45°, and the fourth signal 540 has a phase of 90°. When an MR spectrum is generated by using the MR signal including the first signal 510, the second signal 520, the third signal 530, and the fourth signal 540, it may be difficult to accurately measure amounts of a metabolite corresponding to the second signal 520 and a metabolite corresponding to the fourth signal 540. The signal control unit 450 may select a reference signal from the first signal 510, the second signal 520, the third signal 530, and the fourth signal 540 extracted from the MR signal. For example, the signal control unit 450 may select the first signal 510 having a phase of 0° as a reference signal.

The signal control unit 450 may adjust a phase of a signal which is different from a phase of the first signal 510 that is selected as a reference signal. That is, the signal control unit 450 may adjust phases of the second signal 520 and the fourth signal 540 which are different from the phase of the first signal 510. The signal control unit 450 may adjust the phases of the second signal 520 and the fourth signal 540 so that the phases of the second signal 520 and the fourth signal 540 are the same as the phase of the first signal 510. Alternatively, the signal control unit 450 may adjust the phases of the second signal 520 and the fourth signal 540 so that a phase difference between the phases of the second signal 520 and the fourth signal 540 and the phase of the first signal 510 is within a predetermined range. The signal control unit 450 may adjust the phases of the second signal 520 and the fourth signal 540 by time-delaying the second signal 520 and the fourth signal 540.

FIG. 6 shows a graph illustrating a plurality of signals including a signal with phase adjusted in the time domain. A phase of a phase adjusted second signal 520' and a phase of a phase adjusted fourth signal 540' are the same as a phase of the first signal 510. The signal control unit 450 may generate a combination signal by combining the first signal 510 and the third signal 530 with the phase adjusted second signal 520' and fourth signal 540', and the spectrum generating unit 470 may generate an MR spectrum of the object based on the combination signal generated by the signal control unit 450. When the MR spectrum of the object is generated by using signals having phases of 0°, shapes of peak areas shown in the MR spectrum may be the same as a shape of the frequency spectrum of FIG. 2A, and thus a metabolite may be accurately quantized. Although the signal control unit 450 may select the fourth signal 540 having a phase of 90° as a reference signal from among the plurality of signals of FIG. 5, when the fourth signal 540 is selected as a reference signal, shapes of peak areas shown in the MR spectrum may be the same as a shape of the frequency spectrum of FIG. 2C, and thus the user may measure an amount of a metabolite after applying a phase offset of 90° to the MR spectrum.

Although the MR spectrum is generated by using the combination signal obtained by combining a specific plurality of signals included in a plurality of frequency ranges, the MR signal emitted from the object may further include other signals indicating information about the object as well as the specific plurality of signals. Accordingly, the signal control unit 450 may obtain a difference signal by subtracting the extracted plurality of signals from the MR signal, and may generate a second combination signal by combining the obtained difference signal with the combination signal. The spectrum generating unit 470 may generate the MR spectrum of the object based on the second combination signal. Thereby, an MR spectrum is generated that includes other signals incorporating information about the object as well as the plurality of signals corresponding to the metabolites selected by the user.

An object metabolite typically comprises mostly water, and the echo MR signal emitted from the object is most affected by a signal emitted from the water. Accordingly, it is desirable to remove the signal that is emitted by the water from the MR signal that is emitted from the object in order to accurately quantize the metabolite. Signal control unit 450 may generate the second combination signal by combining a signal obtained by removing a signal corresponding to a resonance frequency range of water from the difference signal with the combination signal. Signals are combined by signal synchronized linear addition in a digital data processor as known. In another embodiment a weighted signal addition may be employed to minimize water signal contribution, for example.

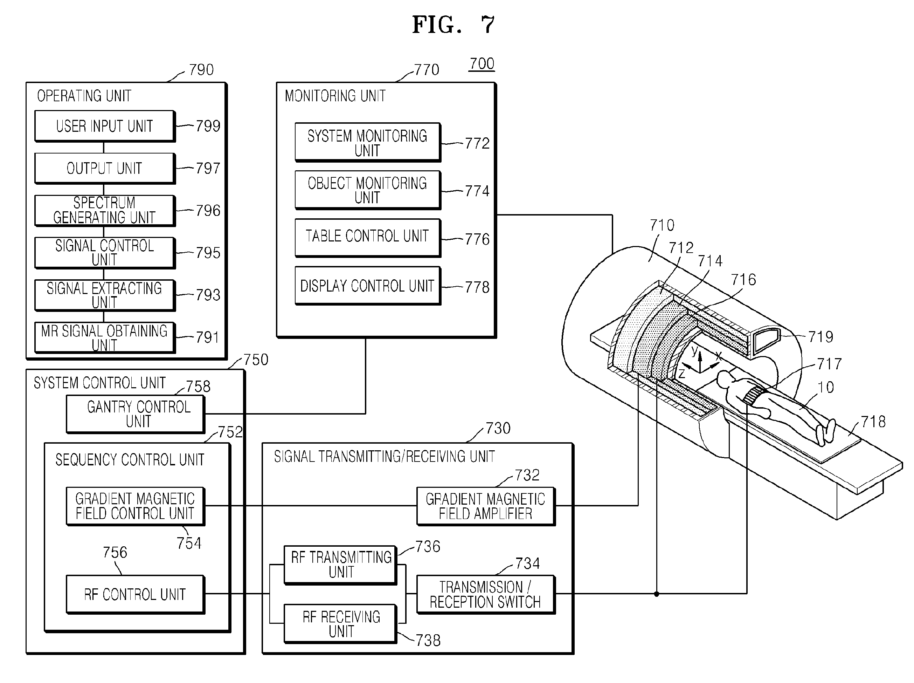

FIG. 7 shows an apparatus 700 for generating an MR spectrum including an MRI system. Apparatus 700 may include a gantry 710, a signal transmitting/receiving unit 730, a system control unit 750, a monitoring unit 770, and an operating unit 790. The gantry 710 blocks electromagnetic waves generated by a main magnet 712, a gradient coil 714, a fixed RF coil 716, and a detachable RF coil 717 from being radiated to the outside. A static magnetic field and a gradient magnetic field are formed in a bore in the gantry 710, and an RF signal is emitted to an object 10 on table 718. The main magnet 712, the gradient coil 714, and the fixed RF coil 716 may be arranged in a predetermined direction on the gantry 710. The predetermined direction may include a coaxial circumferential direction. The object 10 may be placed on a table 718 that may be inserted into a cylinder along a horizontal axis. The main magnet 712 generates a static magnetic field for aligning magnetic dipole moments of atomic nuclei included in the object 10 with a predetermined direction. As the static magnetic field generated by the main magnet 712 is strong and uniform, a relatively precise and accurate MR image of the object 10 may be obtained. The gradient coil 714 may include X, Y, and Z coils that generate gradient magnetic fields in X, Y, and Z-axes that are mutually orthogonal. The gradient coil 714 may induce different resonance frequencies according to different body parts of the object 10 and may provide position information of each of the body parts of the object 10.

The fixed RF coil 716 and the detachable RF coil 717 may emit an RF signal to a patient, and may receive an echo MR signal emitted from the patient. The fixed RF coil 716 may transmit to a patient an RF signal having the same frequency as a frequency of a precession toward an atomic nuclei, and in response to turn off the RF signal detachable RF coil 717 may receive an echo MR signal emitted from the patient. In order to change an atomic nucleus from a low energy state to a high energy state, the fixed RF coil 716 may generate an electromagnetic signal, for example, an RF signal, having an RF corresponding to a type of the atomic nucleus and may apply the electromagnetic signal to the object 10. When the electromagnetic signal generated by the fixed RF coil 716 is applied to the atomic nucleus, the atomic nucleus may be changed from a low energy state to a high energy state. In response to termination of the electromagnetic signal generated by the fixed RF coil 716, the atomic nucleus to which the electromagnetic wave signal has been applied may be changed from a high energy state to a low energy state so that electromagnetic waves having a Larmor frequency may be radiated. Therefore, in response to termination of the electromagnetic wave signal applied to the atomic nucleus, the atomic nucleus may be changed from a high energy state to a low energy state and thus electromagnetic waves having a Larmor frequency may be radiated. The fixed RF coil 716 and the detachable RF coil 717 may receive an electromagnetic wave signal emitted from atomic nuclei in the object 10.

The fixed RF coil 716 and the detachable RF coil 717 may be embodied as one RF transmitting/receiving coil that functions to generate electromagnetic waves having an RF corresponding to a type of atomic nuclei and to receive electromagnetic waves radiated from the atomic nuclei. Alternatively, the fixed RF coil 716 and the detachable RF coil 717 may be respectively embodied as an RF transmitting coil that functions to generate electromagnetic waves having an RF corresponding to a type of atomic nuclei and an RF receiving coil that functions to receive electromagnetic waves radiated from the atomic nuclei. Also, the detachable RF coil 717 may include an RF coil for a body part of the object 10 such as a head RF coil, a breast RF coil, a leg RF coil, a neck RF coil, a shoulder RF coil, a wrist RF coil, or an ankle RF coil. The detachable RF coil 717 may communicate with an external apparatus in a wired and/or wireless manner, and may employ dual tuner communication with variable communication frequency band. Also, the detachable RF coil 717 may include a birdcage coil, a surface coil, and a transverse electromagnetic (TEM) coil structure and may comprise a transmission-only coil, a reception-only coil, and/or a combined transmission/reception coil. Also, the detachable RF coil 717 may include an RF coil with multiple channels such as 16 channels, 32 channels, 72 channels, and 144 channels. The gantry 710 may further include a display unit 719 that is located outside the gantry 710, and a display unit (not shown) that is located inside the gantry 710. Predetermined information may be provided to the user or the object 10 through the display units located inside and outside the gantry 710.

The signal transmitting/receiving unit 730 may control a gradient magnetic field that is formed in a bore of the gantry 710 in response to a predetermined MR sequence, and may control an RF signal and an MR signal to be transmitted/received. The signal transmitting/receiving unit 730 may include a gradient magnetic field amplifier 732, a transmission/reception switch 734, an RF transmitting unit 736, and an MR receiving unit 738. The gradient magnetic field amplifier 732 may drive the gradient coil 714 included in the gantry 710, and may supply to the gradient coil 714 a pulse signal for generating a gradient magnetic field under the control of a gradient magnetic field control unit 754. Gradient magnetic fields in X, Y, and Z-axes may be provided by controlling a pulse signal supplied from the gradient magnetic field amplifier 732 to the gradient coil 714.

The RF transmitting unit 736 and the MR receiving unit 738 are coupled to the fixed RF coil 716 and the detachable RF coil 717. The RF transmitting unit 736 may supply an RF pulse having a Larmor frequency to the fixed RF coil 716 and the detachable RF coil 717, and the MR receiving unit 738 may receive an MR signal received by the fixed RF coil 716 and the detachable RF coil 717. The transmission/reception switch 734 may adjust directions in which an RF signal and an MR signal are transmitted/received. For example, the transmission/reception switch 734 may enable an RF signal to be emitted towards the object 10 by the fixed RF coil 716 and the detachable RF coil 717 during a transmission mode, and may enable an MR signal to be received from the object 10 by the fixed RF coil 716 and the detachable RF coil 717 during a reception mode. The transmission/reception switch 734 may be controlled by a control signal from an RF control unit 756.

The monitoring unit 770 may monitor or control the gantry 710 or devices mounted on the gantry 710. The monitoring unit 770 may include a system monitoring unit 772, an object monitoring unit 774, a table control unit 776, and a display control unit 778. The system monitoring unit 772 may monitor and control a state of a static magnetic field, a state of a gradient magnetic field, a state of an RF signal, a state of an RF coil, a state of the table 718, a state of a device that measures body information of the object 10, a state of a power supply, a state of a heat exchanger, and a state of a compressor. The object monitoring unit 774 monitors a state of the object 10 using a camera for observing a movement or a location of the object 10, a respiration measurement device for measuring a respiration of the object 10, an electrocardiogram (ECG) measurement device for measuring an ECG of the object 10, or a temperature measurement device for measuring a temperature of the object 10, and the respiration measurement device, the ECG measurement device, or the temperature measurement device may be attached to or detached from the gantry 710.

The table control unit 776 controls movement of the table 718 on which the object 10 is placed using sequence control provided by a sequence control unit 752. For example, when a moving image of the object 10 is to be captured, the table control unit 776 may move the table 718 continuously or intermittently according to a sequence control of the sequence control unit 752. Accordingly, the object 10 may be imaged with a field of view (FOV) greater than a FOV of the gantry 710. The display control unit 778 controls display units that are located outside and inside the gantry 710. In detail, the display control unit 778 may control the display units that are located outside and inside the gantry 710 to be turned on/off, or may control a scene to be displayed on the display units. When a speaker is located inside or outside the gantry 710, the display control unit 778 may control the speaker to be turned on/off or a sound to be output through the speaker.

The system control unit 750 may include the sequence control unit 752 that controls a sequence of signals formed in the gantry 710, and a gantry control unit 758 that controls the gantry 710 and devices mounted on the gantry 710. The sequence control unit 752 may include the gradient magnetic field control unit 754 that controls the gradient magnetic field amplifier 732, and the RF control unit 756 that controls the RF transmitting unit 736, the MR receiving unit 738, and the transmission/reception switch 734. The sequence control unit 752 may control the gradient magnetic field amplifier 732, the RF transmitting unit 736, the MR receiving unit 738, and the transmission/reception switch 734 using a pulse sequence received from the operating unit 790. The pulse sequence includes information needed to control the gradient magnetic field amplifier 732, the RF transmitting unit 736, the MR receiving unit 738, and the transmission/reception switch 734, for example, information about an MR signal intensity, an application time, and an application timing of a pulse signal applied to the gradient coil 714.

The operating unit 790 may give pulse sequence information to the system control unit 750, and may control an overall operation of the apparatus 700. The operating unit 790 may include an MR signal obtaining unit 791, a signal extracting unit 793, a signal control unit 795, a spectrum generating unit 796, an output unit 797, and a user input unit 799. The MR signal obtaining unit 791, the signal extracting unit 793, and the signal control unit 795 operation is described. The spectrum generating unit 796 may generate MR image data of the object 10 by processing an MR signal received from the MR receiving unit 738. The spectrum generating unit 796 performs different signal processing functions such as amplification, frequency conversion, phase detection, low frequency amplification, and filtering on the MR signal received by the MR receiving unit 738. The spectrum generating unit 796 may store digital data in, for example, a k-space (for example, called a Fourier space or a frequency space) of a memory, and may reconstruct the digital data into image data through 2D or 3D Fourier transform. Also, the spectrum generating unit 796 may perform synthesis or differential operation on the image data, if necessary. Examples of the synthesis may include pixel addition and maximum intensity projection (MIP). Also, the spectrum generating unit 796 may store in the memory (not shown) or an external server, the reconstructed image data and the image data on which the synthesis or the differential operation has been performed. Also, the different signal processing functions performed on the MR signal by the spectrum generating unit 796 may be performed in parallel. For example, a plurality of MR signals received by a multi-channel RF coil may be reconstructed into image data by applying parallel signal processing functions on the plurality of MR signals.

The spectrum generating unit 796 may generate an MR spectrum of the object 10 based on a combination signal or a second combination signal generated by the signal control unit 795. The output unit 797 may output the image data generated or reconstructed by the spectrum generating unit 796 to the user. Also, the output unit 797 may output information needed for the user to manipulate the apparatus 700 such as user interface (UI) information, user information, or object information. The output unit 797 may include a speaker, a printer, a cathode-ray tube (CRT) display unit, an liquid crystal display (LCD) display unit, a plasma display panel (PDP) display unit, an organic light-emitting display (OLED) unit, a field emission display (FED) unit, a light-emitting diode (LED) display unit, a vacuum fluorescent display (VFD) unit, a digital light processing (DLP) display unit, a primary flight display (PFD) unit, a 3D display unit, and a transparent display unit.

The user may input object information, parameter information, or information about scan conditions, a pulse sequence, image synthesis, or a differential operation. Also, the user may select a specific metabolite to be observed by using the user input unit 799. The user input unit 799 may include a keyboard, a mouse, a trackball, a voice recognition unit, a gesture recognition unit and a touch screen, for example. Although the signal transmitting/receiving unit 730, the monitoring unit 770, the system control unit 750, and the operating unit 790 are separated from one another in FIG. 7, it will be understood by one of ordinary skill in the art that functions performed by the signal transmitting/receiving unit 730, the monitoring unit 770, the system control unit 750, and the operating unit 790 may be performed by another element. For example, although the spectrum generating unit 796 converts an MR signal received by the MR receiving unit 738 into a digital signal, the conversion to the digital signal may be directly performed by the MR receiving unit 738, the fixed RF coil 716, or the detachable RF coil 717.

The gantry 710, the fixed RF coil 716, the detachable RF coil 717, the signal transmitting/receiving unit 730, the monitoring unit 770, the system control unit 750, and the operating unit 790 may be connected to one another in a wired or wireless manner. When the gantry 710, the fixed RF coil 716, the detachable RF coil 1717, the signal transmitting/receiving unit 730, the monitoring unit 770, the system control unit 750, and the operating unit 790 are connected in a wireless manner, an apparatus (not shown) for synchronizing clock signals therebetween may be further included. Communication between the gantry 710, the fixed RF coil 716, the detachable RF coil 717, the signal transmitting/receiving unit 730, the monitoring unit 770, the system control unit 750, and the operating unit 790 may be high speed digital interface communication such as low voltage difference signaling (LVDS), asynchronous serial communication such as universal asynchronous receiver transmitter (UART), low latency network protocol such as controller area network (CAN), or optical communication, for example.

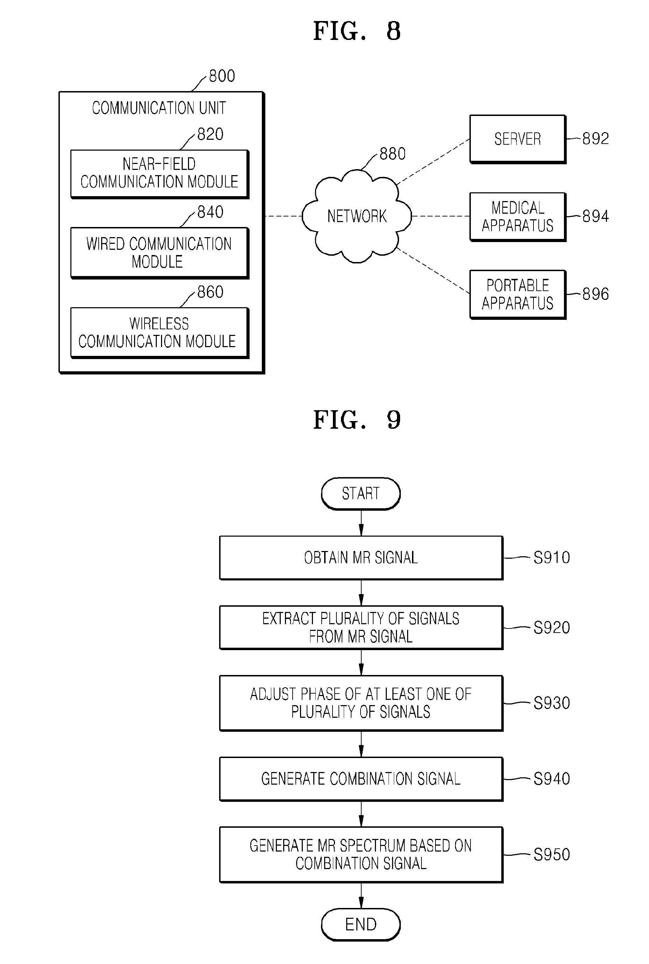

FIG. 8 shows a communication unit 800 employed by the apparatus 700 of FIG. 7 that may be connected to at least one of the gantry 710, the signal transmitting/receiving unit 730, the monitoring unit 770, the system control unit 750, and the operating unit 790 of FIG. 7. Unit 800 may be located in one or more of the units of system 700. The communication unit 800 may transmit and receive data to and from a hospital server or another medical apparatus in a hospital connected through a picture archiving and communication system (PACS), and may communicate data according to a digital imaging and communications in medicine (DICOM) standard. Unit 800 may be connected to a network 880 in a wired or wireless manner, and may communicate with an external server 892, an external medical apparatus 894, or an external portable apparatus 896.

Unit 800 may transmit/receive data related to diagnosis of the object 10 through the network 880, and may transmit/receive a medical image obtained by the external medical apparatus 896 such as a CT apparatus, an MRI apparatus, or an X-ray apparatus. Furthermore, the communication unit 800 may receive a diagnosis history or a treatment schedule of a patient from the server 892 and may use the received diagnosis history or treatment schedule to diagnose the object 10. Also, the communication unit 800 may perform data communication with the server 892 and the external medical apparatus 894 in the hospital and with the external portable apparatus 896 such as a mobile phone, a personal digital assistant (PDA), or a notebook computer of a doctor or a client. Unit 800 may transmit to a user through the network 880, information about whether the apparatus 700 is abnormal or medical image quality information, and may receive feedback from the user.

The communication unit 800 may include one or more elements that may communicate with an external apparatus, for example, a near-field communication module 820, a wired communication module 840, and a wireless communication module 860. The near-field communication module 820 refers to a module that performs near-field communication with a device within a predetermined distance. Examples of a near-field communication technology according to an embodiment of the present invention may include, but are not limited to, a wireless local area network (LAN), Wi-Fi, Bluetooth, Zigbee, Wi-Fi Direct (WFD), ultra wideband (UWB), infrared data association (IrDA), Bluetooth low energy (BLE), and near-field communication (NFC). The wired communication module 840 refers to a module for performing communication using an electrical signal or an optical signal. Examples of wired communication may include use of a pair cable, a coaxial cable and an optical fiber cable, for example. The wireless communication module 860 transmits/receives a wireless signal to/from at least one of a base station, an external apparatus, and a server in a mobile communication network. The wireless signal may include a voice call signal, a video call signal, and different types of data according to text/multimedia message transmission/reception.

FIG. 9 shows a flowchart of a method of generating an MR spectrum including operations sequentially performed by the apparatus 400 of FIG. 4. Accordingly, although omitted, the description made for the apparatus 400 of FIG. 4 may apply to the method of FIG. 9.

In operation S91 0, the apparatus 400 obtains an echo MR signal that is emitted from an object in response to an RF signal that is emitted towards the object. The apparatus 400 may obtain an MR signal that is received by an RF coil. In operation S920, the apparatus 400 extracts a plurality of signals respectively corresponding to a plurality of frequency ranges from the MR signal. Each of the plurality of frequency ranges may correspond to each of resonance frequency ranges of a plurality of metabolites selected by a user. The apparatus 400 may extract the plurality of signals by applying an SVD algorithm to the MR signal in a time domain. In operation S930, the apparatus 400 adjusts a phase of at least one of the plurality of signals in a time domain. The apparatus 400 may adjust a phase of a second signal having a phase that is different from a phase of a first signal from among the plurality of signals. For example, the apparatus 400 may determine a signal having a phase of 0° from among the plurality of signals as the first signal, and may adjust a phase of the second signal so that the phase of the second signal which is different from a phase of the first signal is the same as the phase of the first signal.

In operation 940, the apparatus 400 generates a combination signal by combining the plurality of signals including the at least one phase adjusted signal. The apparatus 400 may generate a second combination signal by combining a difference signal obtained by subtracting the extracted plurality of signals from the MR signal with the combination signal. In operation S950, the apparatus 400 generates an MR spectrum of the object based on the combination signal. The apparatus 400 may output the generated MR spectrum through a display unit.

The afore-described embodiments may be implemented as an executable program, and may be executed by a general-purpose digital computer that runs the program by using a computer-readable recording medium. Examples of the computer-readable medium are a magnetic recording medium (a read-only memory (ROM), a floppy disc, a hard disc, etc.), and an optical recording medium (a compact disk (CD)-ROM, a digital versatile disk (DVD), etc.). While the system has been particularly shown and described with reference to exemplary embodiments thereof, it will be understood by one of ordinary skill in the art that different changes in form and details may be made therein.

The above-described embodiments can be implemented in hardware, firmware or via the execution of software or computer code that can be stored in a recording medium such as a CD ROM, a Digital Versatile Disc (DVD), a magnetic tape, a RAM, a floppy disk, a hard disk, or a magneto-optical disk or computer code downloaded over a network originally stored on a remote recording medium or a non-transitory machine readable medium and to be stored on a local recording medium, so that the methods described herein can be rendered via such software that is stored on the recording medium using a general purpose computer, or a special processor or in programmable or dedicated hardware, such as an ASIC or FPGA. As would be understood in the art, the computer, the processor, microprocessor controller or the programmable hardware include memory components, e.g., RAM, ROM, Flash, etc. that may store or receive software or computer code that when accessed and executed by the computer, processor or hardware implement the processing methods described herein. In addition, it would be recognized that when a general purpose computer accesses code for implementing the processing shown herein, the execution of the code transforms the general purpose computer into a special purpose computer for executing the processing shown herein. The functions and process steps herein may be performed automatically or wholly or partially in response to user command. An activity (including a step) performed automatically is performed in response to executable instruction or device operation without user direct initiation of the activity. No claim element herein is to be construed under the provisions of 35 U.S.C. 112, sixth paragraph, unless the element is expressly recited using the phrase "means for."

**Claims**

1. A method of generating a magnetic resonance (MR) spectrum, the method comprising:

    obtaining an MR echo signal from an object in response to a transmitted radio frequency (RF) signal that is emitted towards the object; extracting from the MR signal, a plurality of signals corresponding to a plurality of frequency ranges; adjusting a phase of at least one of the extracted plurality of signals in a time domain; generating a combination signal by combining the plurality of signals including the at least one extracted phase adjusted signal; and generating an MR spectrum of the object in response to the combination signal.

2. The method of claim 1, wherein each of the plurality of frequency ranges individually correspond to individual resonance frequency ranges of a plurality of metabolites selected by a user.

3. The method of claim 1, wherein the extracting comprises extracting the plurality of signals by applying a singular value decomposition (SVD) algorithm to the MR signal in the time domain.

4. The method of claim 1, wherein the adjusting of the phase of the at least one of the plurality of signals comprises adjusting a second signal having a phase that is different from a phase of a first signal from among the plurality of signals in the time domain by time delaying the second signal relative to the first signal.

5. The method of claim 4, wherein the adjusting of the phase of the second signal comprises adjusting the phase of the second signal so that a phase difference between the phase of the first signal and the phase of the second signal lies within a predetermined range.

6. The method of claim 4, wherein the adjusting of the phase of the second signal comprises adjusting the phase of the second signal so that the phase of the second signal is substantially the same as the phase of the first signal.

7. The method of claim 4, wherein the adjusting of the phase of the at least one of the plurality of signals comprises selecting from the plurality of signals, a signal having a phase of 0° as the first signal.

8. The method of claim 1, further comprising:

generating a second combination signal by combining a difference signal obtained by removing the extracted plurality of signals from the MR signal with the combination signal; and generating the MR spectrum of the object based on the second combination signal.

9. The method of claim 8, wherein the generating of the second combination signal comprises generating the second combination signal by combining a signal obtained by removing a signal corresponding to a resonance frequency range of water from the difference signal with the combination signal.

10. The method of claim 1, further comprising generating the combination signal in the time domain, converting the combination signal to the frequency domain and generating the MR spectrum from the combination signal in the frequency domain and outputting the generated MR spectrum.

11. A computer-readable recording medium having embodied thereon a program for executing the method of claim 1.

12. An apparatus for generating a magnetic resonance (MR) spectrum, the apparatus comprising:

an MR signal acquisition unit that obtains an MR echo signal from an object in response to a transmitted radio frequency (RF) signal that is emitted towards the object; a signal extracting unit that extracts from the MR signal, a plurality of signals corresponding to a plurality of frequency ranges; a signal control unit that adjusts a phase of at least one of the extracted plurality of signals in a time domain, and generates a combination signal by combining the plurality of signals including the at least one extracted phase adjusted signal; and

a spectrum generating unit that generates an MR spectrum of the object in response to the combination signal.

13. The apparatus of claim 12, wherein each of the plurality of frequency ranges individually correspond to individual resonance frequency ranges of a plurality of metabolites selected by a user.

14. The apparatus of claim 12, wherein the signal control unit adjusts a phase of a second signal having a phase that is different from a phase of a first signal from among the plurality of signals in the time domain by time delaying the second signal relative to the first signal.

15. The apparatus of claim 12, further comprising an output unit that outputs the generated MR spectrum.

# FIG. 1

# FIG. 2

FIG. 3A

FIG. 3B

FIG. 3C

# FIG. 4

400

410
430

MR SIGNAL
OBTAINING UNIT

SIGNAL
EXTRACTING UNIT

450
470

SIGNAL
CONTROL UNIT

SPECTRUM
GENERATING UNIT

# FIG. 5

# FIG. 6

# FIG. 7

**790 — OPERATING UNIT**
- USER INPUT UNIT — 799
- OUTPUT UNIT — 797
- SPECTRUM GENERATING UNIT — 796
- SIGNAL CONTROL UNIT — 795
- SIGNAL EXTRACTING UNIT — 793
- MR SIGNAL OBTAINING UNIT — 791

**770 — MONITORING UNIT**      **700**
- SYSTEM MONITORING UNIT — 772
- OBJECT MONITORING UNIT — 774
- TABLE CONTROL UNIT — 776
- DISPLAY CONTROL UNIT — 778

**750 — SYSTEM CONTROL UNIT**
- 758 — GANTRY CONTROL UNIT
- 752 — SEQUENCY CONTROL UNIT
  - 754 — GRADIENT MAGNETIC FIELD CONTROL UNIT
  - 756 — RF CONTROL UNIT

**730 — SIGNAL TRANSMITTING/RECEIVING UNIT**
- 732 — GRADIENT MAGNETIC FIELD AMPLIFIER
- 736 — RF TRANSMITTING UNIT
- 738 — RF RECEIVING UNIT
- 734 — TRANSMISSION / RECEPTION SWITCH

710, 712, 714, 716, 719, 717, 10, 718

# FIG. 8

COMMUNICATION UNIT — 800

NEAR-FIELD COMMUNICATION MODULE — 820

WIRED COMMUNICATION MODULE — 840

WIRELESS COMMUNICATION MODULE — 860

NETWORK — 880

SERVER — 892

MEDICAL APPARATUS — 894

PORTABLE APPARATUS — 896

# FIG. 9

START

OBTAIN MR SIGNAL — S910

EXTRACT PLURALITY OF SIGNALS FROM MR SIGNAL — S920

ADJUST PHASE OF AT LEAST ONE OF PLURALITY OF SIGNALS — S930

GENERATE COMBINATION SIGNAL — S940

GENERATE MR SPECTRUM BASED ON COMBINATION SIGNAL — S950

END

Europäisches
Patentamt

European
Patent Office

Office européen
des brevets

# EUROPEAN SEARCH REPORT

Application Number

EP 14 15 3816

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HU H ET AL: "Reference Deconvolution, Phase Correction, and Line Listing of NMR Spectra by the 1D Filter Diagonalization Method", JOURNAL OF MAGNETIC RESONANCE, ACADEMIC PRESS, ORLANDO, FL, US, vol. 134, no. 1, 1 September 1998 (1998-09-01), pages 76-87, XP004407875, ISSN: 1090-7807, DOI: 10.1006/JMRE.1998.1516 * the whole document * | 1-15 | INV. G01R33/46 |
| X | DERBY K ET AL: "BASELINE DECONVOLUTION, PHASE CORRECTION, AND SIGNAL QUANTIFICATION IN FOURIER LOCALIZED SPECTROSCOPIC IMAGING", MAGNETIC RESONANCE IN MEDICINE, JOHN WILEY & SONS, INC, US, vol. 12, no. 2, 1 November 1989 (1989-11-01), pages 235-240, XP000084358, ISSN: 0740-3194 * the whole document * | 1-15 | |
| X | R.BARTHA ET AL: "Comparing the Quantification of Single Volume and CSI Short Echo STEAM 1H Spectra in the Left Basal Ganglia", PROC.INTL.SOC.MAG.RESON.MED., vol. 2, 27 April 1996 (1996-04-27), page 1182, XP002730100, * the whole document * | 1-15 | TECHNICAL FIELDS SEARCHED (IPC)  G01R |

-/--

The present search report has been drawn up for all claims

| Place of search | Date of completion of the search | Examiner |
|---|---|---|
| Munich | 24 September 2014 | Lersch, Wilhelm |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 14 15 3816

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | A. A. DE GRAAF ET AL: "Quality: quantification improvement by converting lineshapes to the lorentzian type", MAGNETIC RESONANCE IN MEDICINE, vol. 13, no. 3, 1 March 1990 (1990-03-01), pages 343-357, XP055142139, ISSN: 0740-3194, DOI: 10.1002/mrm.1910130302 * the whole document * | 1-15 | |
| Y | PIJNAPPEL W W F ET AL: "SVD-BASED QUANTIFICATION OF MAGNETIC RESONANCE SIGNALS", JOURNAL OF MAGNETIC RESONANCE, ACADEMIC PRESS, ORLANDO, FL, US, vol. 97, no. 1, 1 March 1992 (1992-03-01), pages 122-134, XP000615198, ISSN: 1090-7807 * the whole document * | 1-15 | |
| Y | YANN LE FUR ET AL: "FID modulus: a simple and efficient technique to phase and align MR spectra", MAGMA MAGNETIC RESONANCE MATERIALS IN PHYSICS, BIOLOGY AND MEDICINE, vol. 27, no. 2, 21 June 2013 (2013-06-21), pages 131-148, XP055142144, ISSN: 0968-5243, DOI: 10.1007/s10334-013-0381-8 * see pages 132-134,138,141-147 * | 1-15 | TECHNICAL FIELDS SEARCHED (IPC) |

-/--

The present search report has been drawn up for all claims

| Place of search | Date of completion of the search | Examiner |
|---|---|---|
| Munich | 24 September 2014 | Lersch, Wilhelm |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 2 821 804 A1**

Europäisches
Patentamt
European
Patent Office
Office européen
des brevets

**EUROPEAN SEARCH REPORT**

Application Number

EP 14 15 3816

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | JOHNSON G: "SELF-CORRECTION OF PROTON SPECTROSCOPIC IMAGES FOR GRADIENT EDDY CURRENT DISTORTIONS AND STATIC FIELD INHOMOGENEITIES", MAGNETIC RESONANCE IN MEDICINE, JOHN WILEY & SONS, INC, US, vol. 30, no. 2, 1 August 1993 (1993-08-01), pages 255-261, XP000385360, ISSN: 0740-3194 * the whole document * | 1-15 | |
| Y | RIDDLE W R ET AL: "REMOVING EFFECTS OF EDDY CURRENTS IN PROTON MR SPECTROSCOPY", MEDICAL PHYSICS, AIP, MELVILLE, NY, US, vol. 19, no. 2, 1 March 1992 (1992-03-01), pages 501-509, XP000896097, ISSN: 0094-2405, DOI: 10.1118/1.596839 * the whole document * | 1-15 | |
| A | R.BARTHA ET AL.: "A Comparison Between Time and Frequency Domain Quantification of Short Echo 1H MR Spectra", PROC.INTL.SOC.MAG.RESON.MED., 19 August 1995 (1995-08-19), page 1946, XP002730101, * the whole document * | 1-15 | TECHNICAL FIELDS SEARCHED (IPC) |
| A | A VAN DEN BOOGAART ET AL: "Time and Frequency Domain Analysis of NMR Data Compared: An Application to 1D 'H Spectra of Lipoproteins", MAGNETIC RESONANCE IN MEDICINE, vol. 31, 1 January 1994 (1994-01-01), pages 347-358, XP055142151, * the whole document * | 1-15 | |

-/--

The present search report has been drawn up for all claims

| Place of search | Date of completion of the search | Examiner |
|---|---|---|
| Munich | 24 September 2014 | Lersch, Wilhelm |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 14 15 3816

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | C.P.HESS ET AL.: "A New Method for Automatic Correction of Phase and Baseline Artifacts in MR Spectroscopic Imaging Data", PROC.INTL.SOC.MAG.RESON.MED., 1993, page 687, XP002730102, * the whole document * | 1-15 | |
| A | CANET D ET AL: "Accurate determination of parameters for <17>O in natural abundance by Fourier transform NMR", JOURNAL OF MAGNETIC RESONANCE, ACADEMIC PRESS, LONDON, GB, vol. 22, no. 3, 1 June 1976 (1976-06-01), pages 537-542, XP023960637, ISSN: 0022-2364, DOI: 10.1016/0022-2364(76)90017-2 [retrieved on 1976-06-01] * the whole document * | 1-15 | |
| A | POULLET J B ET AL: "MRS signal quantitation: A review of time- and frequency-domain methods", JOURNAL OF MAGNETIC RESONANCE, ACADEMIC PRESS, ORLANDO, FL, US, vol. 195, no. 2, 1 December 2008 (2008-12-01), pages 134-144, XP025626033, ISSN: 1090-7807, DOI: 10.1016/J.JMR.2008.09.005 [retrieved on 2008-09-11] * the whole document * | 1-15 | TECHNICAL FIELDS SEARCHED (IPC) |

The present search report has been drawn up for all claims

| Place of search | Date of completion of the search | Examiner |
|---|---|---|
| Munich | 24 September 2014 | Lersch, Wilhelm |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

**EP 2 821 804 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020130079038